# EUROPEAN PATENT SPECIFICATION

(11) **EP 2 698 174 B1**
(45) Date of publication and mention of the grant of the patent: **28.06.2017**
(21) Application number: 13180622.6
(22) Date of filing: 16.08.2013
(51) Int. Cl.: A61M 1/00, A61L 31/00

(54) **Flocked medical tube**
Beflockter medizinischer Schlauch
Tube médical floqué

(30) Priority: 17.08.2012 DE 102012214640
(43) Date of publication of application: 19.02.2014
(73) Proprietor: AESCULAP AG, 78532 Tuttlingen (DE)
(72) Inventor: Odermatt, Erich, 8200 Schaffhausen (CH); Abele, Wolfgang, Dr., 78532 Tuttlingen (DE)
(74) Representative: Patentanwälte Ruff, Wilhelm, Beier, Dauster & Partner mbB

(56) References cited:
- EP-A1- 2 567 715
- EP-A2- 0 315 305
- WO-A1-2010/127875
- WO-A1-2012/018917
- WO-A1-2012/087376
- DE-A1-102011 055 782
- US-A- 2 927 584

## Description

### Field of application and prior art

The invention relates to a medical tube with a flock material, a process for producing said tube and a drainage system.

Medical drainage tubes for vacuum therapy of wounds are known. Tubes of this kind are described for example in WO 2006/005939 A1 and WO 2009/140376 A1. EP0315305 discloses a male external catheter wherein its surface comprises a thin layer of adhesive and flock material. A negative pressure wound treatment system comprising a component being made of a felted or flocked fibrous material is known from WO 2012/087376 A1. A cannular device for attaching to a chamber of the heart and having flocking is known from WO 2012/018917 A1.

Drainage tubes are used in endoluminal vacuum therapy, usually together with a sponge, which is provided for absorbing body fluids, for example wound fluids.

This is, however, associated with some disadvantages.

Thus, the sponge together with the tube must as a rule be changed every three days, as otherwise there is a risk of tissue adhesions, which makes it difficult to remove the sponge, cause injuries and moreover can be painful for the patient.

In addition, as wound healing progresses, the sponge size must as a rule be adjusted, which on removal can lead to a (re-) opening-up of a wound.

Another disadvantage is that passage of the sponge can prove difficult, especially through small body orifices. An additional complication is that the sponges usually employed often only possess moderate to poor sliding properties along tissue surfaces.

Another problem relates to the handling of the sponge. Depending on the material of the sponge, its structural integrity can be destroyed if for example it is grasped and moved with a guiding instrument, for example forceps.

In addition, the generally open-pore configuration of the sponge can be a disadvantage, as there is always a basic risk of clogging of the pores and a consequent decrease in absorption capacity of the sponge. It may therefore even be necessary to use several sponges.

Another possible problem is the joint between sponge and tube. Usually the sponge is glued to the tube. This can result in undesirable thickening in the region of the glued points, which can impair fluid communication between sponge and tube.

Finally, the sponge together with the tube must usually be trimmed specifically for the patient.

### The problem and its solution

Against this background, the problem to be solved by the present invention was therefore to provide a technical solution that addresses the problems mentioned above.

This problem is solved with a medical drainage tube with the features of independent claim 1, with a process for producing a medical tube according to claim 9 and with a drainage system with the features of claim 10. Preferred embodiments of the tube are defined in claims 2 to 8. The wording of all claims is incorporated herewith by explicit reference to the contents of the description.

In a first aspect, the invention relates to a medical drainage tube as claimed in claim 1. The tube is intended in particular for use in vacuum therapy, preferably endoluminal vacuum therapy.

The tube according to the invention comprises at least one tube body and is characterized in particular in that moreover it has a flock material. In other words, the present invention provides a flocked tube for use in medicine.

The medical tube of the present invention is a medical drainage tube and correspondingly the at least one tube body is at least one drainage tube body.

In other words the tube according to the invention is a tube for leading away or aspirating especially pathological and/or increased body fluids (drainage).

The term "at least one drainage tube body", in the sense of the present invention, comprises a drainage tube body or a plurality of drainage tube bodies, i.e. two or more drainage tube bodies.

The tube body of the tube according to the invention preferably defines a dimensionally stable lumen or a dimensionally stable cavity, which is bounded by a wall of the tube body. This has the advantage that on applying a negative pressure or vacuum, collapse of the tube body can be avoided.

The ends of the tube body are preferably configured open.

However, it can also be envisaged according to the invention that one of the ends, preferably an end of the tube body that is flocked, i.e. has the flock material, is sealed, for example glued or welded. This can be of advantage mainly in the case of a drainage tube, for increasing its suction power.

Furthermore, it can be envisaged according to the invention that the tube body does not define a lumen or a cavity until a medium flows through it, in particular a fluid, for example rinsing fluid, disinfecting fluid, wound fluid etc. or it is supplied with a gas, for example compressed air. In other words the tube body can originally be of flat configuration.

The term "flock material" is to be understood, in the sense of the present invention, as a material that can be applied on the surface of the tube body by means of a flocking technique using an adhesive.

The tube according to the invention makes the use of a sponge, especially of the type described at the beginning, superfluous, so that the disadvantages described in the introduction in connection with sponges can be avoided.

Thus, in particular, the tube according to the invention allows a prolonged residence time in a patient's body, because (in contrast to the case of a sponge) there are no undercuts or pores, into which the body tissue could grow, with formation of tissue adhesions.

The risk of tissue adhesions can moreover be minimized because as a rule the flock material offers less area for the growth or ingrowth of cells or tissues and in particular exerts a separating function against cells or tissues. In addition, the flock material can be selected on the basis that, owing to its flexibility, it can be pulled off of a tissue more easily.

Another advantage is that the tube according to the invention can have much smaller dimensions than the sponges known from the prior art. Therefore the tube can also be moved through small body orifices without major problems and in particular without aids.

Furthermore, placement of the tube according to the invention is possible by means of much smaller trocars and/or incisions. As a result, placement and removal of the tube is simplified considerably and moreover is less traumatic for the patient.

For filling larger body lumina or cavities, there is in addition the possibility of transforming the tube according to the invention into a ball following its placement in a body lumen or cavity.

A further advantage is in particular that the flock material increases the surface area of the tube, so that much less tube length has to be pushed into a body lumen or a body cavity.

Another advantage is that as a rule the flock material is mechanically more stable than a sponge, so that the risk of mechanical damage, for example on gripping with forceps, is greatly reduced.

As the flocked tube, in contrast to the sponges known from the prior art, does not have any cavities in the sense of pores, the risk of clogging is also greatly reduced.

Another advantage is that the flock material can endow the tube according to the invention with better sliding properties overall, so that the use of lubricating gels, especially when using small trocars or overtubes, is no longer necessary and direct placement can take place using forceps or in the endoscope working channel.

Depending on the flock material and/or its depth of penetration into the adhesive layer formed on the surface of the tube body, the properties, especially mechanical properties and/or sliding properties, of the tube can be adapted in a targeted manner, i.e. specifically for the indication and/or for the patient.

A further advantage is that, in contrast to gluing a sponge to a tube body, flocking of the tube body can be automated.

Finally, the flocked tube can be tailored or manufactured simply and comfortably with respect to a patient's specific needs.

The flock material is joined via an adhesive layer to the tube body or to the surface, especially inside and/or outside surface, preferably outside surface, of the tube body. Joining is as a rule based on adhesive forces.

When the surface of the tube body is referred to in the following, this is to be understood as the inside and/or outside surface, preferably the outside surface, of the tube body.

The flock material is joined to the tube body or its surface via an adhesive layer at least partially covering the surface of the tube body.

The adhesive layer can cover the surface of the tube body basically completely or on the whole area, apart from any openings in the wall of the tube body.

It is, however, preferable according to the invention if the adhesive layer covers the surface of the tube body only partially or on a partial area.

In particular, the adhesive layer can cover the surface of at least one, especially of only one, end of the tube body.

The adhesive layer can cover the surface of the tube body, preferably starting from at least one end of the tube body, over a tube body length from 5 mm to 500 mm, especially 10 mm to 150 mm, preferably 20 mm to 90 mm.

As a rule the flock material penetrates partially, especially only partially, into the adhesive layer.

Preferably, when an adhesive layer is present that covers the surface of the tube body at least partially, the flock material does not penetrate into the tube body or its wall.

The flock material can penetrate into the adhesive layer over a length of 0.5% to 95%, especially 3% to 50%, preferably 5% to 20%, relative to its total length.

The adhesive layer preferably is not a textile layer or coating. For example, the adhesive layer can be in the form of a film, a foil, a gel, especially hydrogel, or a paste.

In an alternative embodiment, the adhesive layer is of a textile configuration. It is conceivable for example for the adhesive layer to be in the form of a fibre or a filament or else in the form of a large number of fibres or filaments (multifilament). In particular, the adhesive layer can be a tubular textile fabric, especially plaited tube or knitted tube, which envelops or covers the tube body. The tubular fabric can be flocked before or after fitting on the tube body (in the latter case together with the tube body). The embodiments described in this paragraph have the particular advantage that the tube body can be removed again without the tubular fabric from a patient's body, especially in the case when the tubular fabric is formed from an absorbable material.

In another embodiment, the adhesive layer has a proportion of 0.1 wt% to 90 wt%, especially 0.5 wt% to 50 wt%, preferably 1 wt% to 25 wt%, relative to the total weight of the tube.

The adhesive layer can moreover have a thickness between 1 µm and 500 µm, especially 3 µm and 300 µm, preferably 5 µm and 100 µm.

Basically the adhesive layer can be configured to be absorbable, partially absorbable or non-absorbable. In other words the adhesive layer can have an absorbable, partially absorbable or non-absorbable material, preferably polymer, especially copolymer, or can be formed from such a material, preferably polymer, especially copolymer.

The term "copolymer" means, in the sense of the present invention, a polymer that is composed of at least two different monomer units. Therefore the term "copolymer" can cover not only copolymers in the narrower sense, i.e. so-called bipolymers (polymers that are composed of two different monomer units), but in addition terpolymers, tetrapolymers etc. The copolymer can moreover be selected from the group consisting of random copolymer, alternating copolymer, block copolymer or segmented copolymer, graft copolymer and mixtures thereof.

All materials that are suitable for carrying out flocking, i.e. for joining the flock material to the adhesive layer and thus to the surface of the tube body, may basically be considered for the adhesive layer.

Thus, the adhesive layer can basically have a synthetic or industrial polymer and/or biopolymer, i.e. naturally occurring polymer, or can be formed from such a polymer and/or biopolymer.

For example, the adhesive layer can have a non-absorbable material, especially non-absorbable polymer, or can be formed from such a material, especially polymer, which is preferably selected from the group comprising hot-melt adhesives, polyurethanes, especially thermoplastic polyurethanes, polyvinyl alcohol, polyvinyl acetate, polyvinyl chloride, polyvinyl propionates, polyacrylates, cyanoacrylates, poly-α-olefins, polyurethanes, thermoplastic polyurethanes, thermoplastic polyurethane elastomers, vinyl acetate copolymers, especially modified vinyl acetate copolymers, polyesters, vinylpyrrolidone/vinyl acetate copolymer, polycarbonates, rubber, polyoxymethylene, acrylonitrile-butadiene-styrene copolymer, polyethylene glycol-based hydrogels, resins such as for example synthetic resins, waxes such as for example synthetic waxes and/or beeswaxes and mixtures thereof.

Suitable thermoplastic polyurethane elastomers are for example commercially available under the designations Texin^{®} or Desmopan^{®}.

Preferably the adhesive layer has a hot-melt adhesive or is formed from such an adhesive. Suitable hot-melt adhesives can be selected from the group comprising hot-melt adhesives based on polyamides, based on polyester elastomers, based on polyolefins, especially polyethylene, based on amorphous poly-α-olefins, based on polybutene-1, based on ethylene-vinyl acetate copolymer, based on polyurethane elastomers, based on copolyamide elastomers, based on vinylpyrrolidone-vinyl acetate copolymers and mixtures thereof.

Suitable resins that may be mentioned are for example resins that are selected from the group comprising urethane resins, epoxy resins, polyurethane resins, melanin resins, phenol resins, polyester resins, polyamide resins, vinyl ester resins and mixtures thereof.

Alternatively to or in combination with the non-absorbable materials, especially polymers, described in the preceding embodiments, the adhesive layer can have an absorbable material, especially polymer, or can be formed from such a material, especially polymer, which is preferably selected from the group comprising polyhydroxyalkanoates, polyglycolide or polyglycolic acid, polylactide or polylactic acid, polydioxanone, poly-3-hydroxybutyrate or poly-3-hydroxybutyric acid, poly-4-hydroxybutyrate or poly-4-hydroxybutyric acid, technical polysaccharides, oxidized or non-oxidized polysaccharides, polysaccharides bearing amino groups, polysaccharides bearing aldehyde groups, dextran aldehyde, cellulose, cellulose derivatives, for example alkyl celluloses, methylcellulose, hydroxyalkyl celluloses, hydroxymethylcellulose, hydroxyethylcellulose, hydroxypropylcellulose, carboxyalkyl celluloses, carboxymethylcellulose, copolymers thereof, salts thereof, stereoisomers thereof and mixtures thereof.

Alternatively to or in combination with the materials, especially polymers, stated in the preceding embodiments for the adhesive layer, in a further embodiment this can have a biopolymer or can be formed from a biopolymer, which is preferably selected from the group comprising polysaccharides, mucopolysaccharides, starch, amylose, amylopectin, dextran, dextrin, cellulose, chitin, chitosan, hyaluronic acid, dextran sulphate, heparin, heparan sulphate, chondroitin sulphate, dermatan sulphate, structural proteins, extracellular proteins, fibrous proteins, globular proteins, collagen, gelatin, elastin, reticulin, fibronectin, laminin, fibrin, albumin, copolymers thereof, salts thereof, stereoisomers thereof and mixtures thereof.

Of course, the adhesive layer can also have a mixture of the materials described in the preceding embodiments or can consist of such a mixture.

In another embodiment the adhesive layer can be formed from a material, especially a polymer such as for example a thermoplastic polymer, which has a lower melting point than a material, especially polymer such as thermoplastic polymer for example, from which the tube body is formed. As a result, especially advantageously a specific thermal activation of the adhesive layer can be achieved, without the integrity of the tube body structure being impaired. In particular, the melting point of the material used for making the adhesive layer can be lower than the melting point of the flock material. Suitable materials or polymers for the embodiments described in this paragraph are, in addition to polyethylene and/or polypropylene, basically all industrial melt adhesives or mixtures of melt adhesives. To that extent, therefore, reference is also made expressly to the preceding description.

The flock material is in the form of fibres, especially cut fibres and/or ground fibres.

In other words according to the invention the flock material is flock fibres, especially cut flock fibres and/or ground flock fibres.

In particular, the flock material can be textured and/or non-textured flock fibres.

In another embodiment the flock material is branched flock fibres. The use of branched flock fibres has the advantage that the tube body basically has to be flocked with fewer flock fibres in order to produce a relatively large volume, in particular that fills body lumina or cavities.

The flock material in the form of fibres can have a length of 1 mm to 35 mm, especially 2 mm to 20 mm, preferably 3 mm to 10 mm.

Furthermore, the flock material in the form of fibres can have a diameter from 5 µm to 800 µm, especially 7 µm to 300 µm, preferably 10 µm to 150 µm.

Moreover, the flock material in the form of fibres can have a titre (weight per unit length) from 0.1 dtex to 350 dtex, especially 0.5 dtex to 100 dtex, preferably 1 dtex to 80 dtex. The dimension "dtex" (decitex) means a weight per unit length of 1 g per 10000 m length of the flock material.

In another embodiment, the flock material in the form of fibres can project over a length from 0.01 mm to 14 mm, especially 0.1 mm to 9.0 mm, preferably 0.2 mm to 4.8 mm, from the surface of the tube body, especially from the adhesive layer at least partially covering the surface of the tube body.

Furthermore, the flock material in the form of fibres can protrude perpendicularly or essentially perpendicularly from the surface of the tube body, especially the adhesive layer at least partially covering the surface of the tube body. The expression "essentially perpendicularly" can comprise deviations from right angles by up to 5 degrees.

In an alternative embodiment the flock material in the form of fibres protrudes at an oblique angle from the surface of the tube body, especially the adhesive layer at least partially covering the surface of the tube body, which is greater than 0 degree, but less than 85 degrees.

In another embodiment, the flock material in the form of flock fibres is disposed at a density from 1% to 30%, especially 5% to 25%, preferably 10% to 20%, on the surface of the tube body, especially the adhesive layer at least partially covering the surface of the tube body, relative to the theoretical flock material density (coverage density). The term "theoretical flock material density (coverage density)" means, in the sense of the present invention, a value that corresponds to the square of flock materials arranged seamlessly next to one another in the form of flock fibres on one millimetre divided by the fibre diameter.

In another embodiment the flock material in the form of fibres is disposed at a density from 1 to 2000 fibres/mm², especially 5 to 1000 fibres/mm², preferably 10 to 500 fibres/mm², on the adhesive layer at least partially covering the surface of the tube body.

The flock material can basically, apart from any openings in the wall of the tube body, be disposed on part of the area or on the whole area of the surface of the adhesive layer covering the tube body at least partially. The flock material is disposed on only one of the ends of the tube body, on the adhesive layer at least partially covering the surface of the tube body.

In a further embodiment, the flock material, preferably starting from at least one of the ends of the tube body, is disposed over a tube body length from 5 mm to 1000 mm, especially 5 mm to 500 mm, preferably 10 mm to 150 mm, especially preferably 20 mm to 90 mm, on the adhesive layer at least partially covering the surface of the tube body.

The flock material can in principle be arranged in the form of a pattern or irregularly or randomized on the adhesive layer at least partially covering the surface of the tube body.

Suitable arrangements of flock material (and/or arrangements of adhesive layer) on the adhesive layer at least partially covering the surface of the tube body, can be selected from the group comprising linear arrangement, staggered arrangement, helical or spiral arrangement, meander-like arrangement, serpentine arrangement, sinusoidal arrangement, arrangement as circular rings and combinations thereof. According to the invention it is preferable if the flock material is arranged in spirals or circular rings on the adhesive layer at least partially covering the surface of the tube body.

A helical or spiral arrangement of the flock material can be achieved for example by covering the surface of the tube body with a helically or spirally wound filament, serving as adhesive. Alternatively, a helical or spiral arrangement of the flock material can be achieved by means of plaiting acting as adhesive, the filaments of which are partially formed from a thermoplastic polymer with a lower melting point than other filaments of the plaiting.

The possible arrangements of the flock material described in the preceding embodiments can especially advantageously serve as fixing, monitoring, marking, reference or stop or holding means.

The flock material itself can basically be absorbable, partially absorbable or non-absorbable. In other words the flock material can have an absorbable, partially absorbable or non-absorbable material, preferably polymer, especially copolymer, or can be formed from such a material, preferably polymer, especially copolymer.

Thus, flock material can basically have a synthetic or industrial polymer and/or biopolymer, i.e. naturally occurring polymer, or can be formed from such a polymer and/or biopolymer.

Preferably the flock material has a polymer or is formed from a polymer that is selected from the group comprising polyolefins, polyamides, polyesters, polyurethanes, especially thermoplastic polyurethanes, polyhydroxyalkanoates, polysaccharides, technical polysaccharides, oxidized and non-oxidized polysaccharides, polysaccharides bearing amino groups, polysaccharides bearing aldehyde groups, mucopolysaccharides, proteins, structural proteins, extracellular proteins, fibre proteins, globular proteins, enzymes, antibodies, blood clotting factors, copolymers thereof, salts thereof, stereoisomers thereof and mixtures thereof.

For example the flock material can be selected from the group comprising polyethylene, low-density polyethylene, high-density polyethylene, high-molecular polyethylene, ultra-high-molecular polyethylene, polypropylene, polyethylene terephthalate, polypropylene terephthalate, polybutylene terephthalate, polyacrylonitrile, polyamide 6, polyamide 6-6, polyamide 6-12, polyamide 12, silk, especially rayon or spider silk, polytetrafluorethylene, polyvinylidene difluoride, polytetrafluoropropylene, polyhexafluoropropylene, polyvinyl alcohol, polyglycolide or polyglycolic acid, polylactide or polylactic acid, polydioxanone, poly-3-hydroxybutyrate or poly-3-hydroxybutyric acid, poly-4-hydroxybutyrate or poly-4-hydroxybutyric acid, polytrimethylene carbonate, polycaprolactone, cotton, cellulose, cellulose derivatives such as for example alkyl celluloses, methylcellulose, hydroxyalkyl celluloses, hydroxymethylcellulose, hydroxyethylcellulose, hydroxypropylcellulose, carboxyalkyl celluloses, carboxymethylcellulose, starch, amylose, amylopectin, dextran, dextrin, chitin, chitosan, hyaluronic acid, dextran sulphate, heparin, heparan sulphate, chondroitin sulphate, dermatan sulphate, collagen, gelatin, elastin, reticulin, fibronectin, laminin, fibrin, fibrinogen, albumin, copolymers thereof, salts thereof, stereoisomers thereof and mixtures thereof.

A possible copolymer for the flock material is for example a copolymer of glycolide and lactide, especially in a weight ratio from 9:1 to 1:9, especially 7:3 to 3:7.

A possible terpolymer, especially triblock terpolymer, for the flock material is for example a terpolymer, especially triblock terpolymer, of glycolide, trimethylene carbonate and ε-caprolactone. A terpolymer of this kind is commercially available for example under the designation Mono-syn^{®}.

In another embodiment, the flock material has a material that is swellable or soluble in aqueous liquids, especially aqueous solutions, aqueous suspensions, aqueous buffer solutions, aqueous electrolyte solutions and/or body fluids, or in another embodiment is formed from such a material. A suitable material is preferably selected from the group comprising polyvinyl pyrrolidone, polyvinyl alcohol, polyethylene glycol or polyethylene glycol-based copolymers, viscose, cotton, cellulose, cellulose derivatives such as for example alkyl celluloses, methylcellulose, hydroxyalkyl celluloses, hydroxymethylcellulose, hydroxyethylcellulose, hydroxypropylcellulose, carboxyalkyl celluloses, carboxymethylcellulose, starch, amylose, amylopectin, dextran, dextrin, chitin, chitosan, hyaluronic acid, dextran sulphate, heparin, heparan sulphate, chondroitin sulphate, dermatan sulphate, collagen, gelatin, elastin, reticulin, fibronectin, laminin, fibrin, fibrinogen, albumin, copolymers thereof, salts thereof, stereoisomers thereof and mixtures thereof.

The flock material, in particular a water-soluble flock material, especially as described in the preceding paragraph, is suitable in particular for having pharmaceutical or medical active substances added, which will be discussed in more detail below. These active substances can then especially advantageously be released during therapy. In this way the tube according to the invention can be used as a drug delivery device.

In another embodiment the flock material has a physiologically or biologically active material or is formed from such a material. The expression "physiologically active material" or "biologically active material" means a material which, from the cosmetic and/or medical standpoint, can produce advantageous effects in a patient's body.

In particular, the flock material can have a cosmetic active substance, biological active substance, pharmaceutical or medical active substance or mixtures thereof or can be formed from said active substance or a mixture of said active substances.

For example the flock material can have an active substance or can be formed from an active substance that is selected from the group comprising antimicrobial, especially antibiotic, active substances, active substances promoting wound healing, disinfecting active substances, skin-care active substances, oncologic active substances, scar-treating or scar-preventing active substances, anti-inflammatories, pain-relieving active substances, active substances promoting blood coagulation, growth factors, cell differentiation factors, cell-adhesion factors, cell-recruiting factors, cell receptors, cell binding factors, cytokines, peptides, structural proteins, extracellular proteins such as for example collagen, polysaccharides such as for example hyaluronic acid, oligonucleotides, polynucleotides, DNA, RNA, salts thereof, stereoisomers thereof and mixtures thereof.

In particular the flock material can have an active substance or can be formed from an active substance that is selected from the group comprising FGF (fibroblast growth factor), TGF (transforming growth factor), PDGF (platelet-derived growth factor), EGF (epidermal growth factor), GMCSF (granulocyte-macrophage colony stimulation factor), VEGF (vascular endothelial growth factor), IGF (insulin-like growth factor), HGF (hepatocyte growth factor), IL-1B (interleukin-1-B), IL-8 (interleukin-8), NGF (nerve growth factor), BMP or bone morphogenetic proteins such as for example BMP-1 (bone morphogenetic protein 1) and/or BMP-2 (bone morphogenetic protein 2), salts thereof, stereoisomers thereof and mixtures thereof.

Suitable antimicrobial active substances that may be mentioned are for example active substances that are selected from the group comprising biguanides, polyhexamethylene biguanide (PHMB), triclosan, chlorhexidine, gentamicin, copper, zinc, silver, gold, salts thereof, stereoisomers thereof and mixtures thereof.

A preferred active substance promoting wound healing is for example zinc, especially a zinc pigment. Zinc-containing, especially zinc pigment containing, cellulose fibres are especially preferred.

A preferred scar-treating or scar-preventing active substance is captopril.

A suitable anti-inflammatory is for example the pharmaceutical called ibuprofen.

A suitable pain-relieving active substance that may be mentioned is for example the pharmaceutical called paracetamol.

A suitable active substance promoting blood coagulation is for example para-aminomethylbenzoic acid, available commercially under the designation Pambal^{®}.

Of course, the flock material can also have a mixture of the materials described in the preceding embodiments or can consist of such a mixture.

In another embodiment, the flock material has a proportion from 0.01 wt% to 50 wt%, especially 0.5 wt% to 20 wt%, preferably 1.0 wt% to 10 wt%, relative to the total weight of the tube.

In another embodiment the flock material is a mixture of at least two different flock fibres. The flock fibres are preferably varied with respect to their flock fibre length, their flock fibre diameter, their flexibility or stiffness, their material, their absorbability or non-absorbability, their solubility, their structure and/or functionalization, especially with additives, for example with active substances. Regarding the properties of the flock fibres listed above as examples, reference is made entirely to the description up to now.

It can for example be envisaged according to the invention that the flock material is in the form of a mixture of flock fibres with different stiffness or different flexibility. For example the flock material can be a mixture of flock fibres made from polyamide 6 and flock fibres made from polyvinyl alcohol. Whereas the flock fibres of polyamide 6, which is practically un-swellable in water, preferably serve as spacers (between tissue and tube body) during drainage, the flock fibres of polyvinyl alcohol preferably act as a sliding agent.

Furthermore, the flock material can be a mixture of textured and non-textured flock fibres.

Furthermore it can be envisaged according to the invention that the flock material comprises water-soluble or water-swellable flock fibres in addition to water-insoluble flock fibres, wherein the water-soluble or water-swellable flock fibres preferably are longer than the water-insoluble flock fibres. The water-soluble or water-swellable flock fibres act especially advantageously as a sliding agent, making the additional use of a lubricant unnecessary. Moreover, through the presence of water-soluble or water-swellable flock fibres, the imperviousness of the flock material can be modulated. Cotton and/or viscose fibres are especially preferred. Moreover, regarding suitable materials for the flock fibres described in this paragraph, especially for the water-soluble or water-swellable flock fibres, reference is made entirely to the description so far.

Furthermore it can be envisaged according to the invention that the flock material comprises predetermined breaking points. The predetermined breaking points can be selected from the group comprising mechanical predetermined breaking points, chemical predetermined breaking points and combinations thereof. For example, mechanical predetermined breaking points can be accomplished by thinning out the flock material, in particular if the flock material is present in form of fibres. Chemical predetermined breaking points can be, for example, accomplished by structural changes from crystalline to amorphous structure and vice versa. In general, the presence of predetermined breaking points can facilitate removal of the medical tube from a patient's body and can additionally accelerate resorption of the flock material.

In another advantageous embodiment the tube body is formed from a flexible material, for example silicone. As a result, in particular flexible adjustment of the cross-section of the tube body (and thus of the tube) to its surroundings is possible. Moreover, this facilitates removal of the tube from a patient's body.

The tube body can have an outside diameter from 1 mm to 15 mm, preferably 2 mm to 10 mm, more preferably 3 mm to 8 mm, especially preferably 3 mm to 7 mm, most preferably 4 mm to 6 mm.

Furthermore, the tube body can be of multilumen configuration, i.e. can possess two, three or more lumina (cavities). The lumina can in particular have different cross-sections.

For example the tube body can be of three-lumen configuration, wherein one lumen is provided for applying a negative pressure or vacuum, one lumen for rinsing and one lumen for monitoring or control of a negative pressure or vacuum and/or a temperature. Temperature monitoring can provide measurement or monitoring of the inflammatory state and therefore the progress of healing.

In particular, the tube according to the invention can comprise several tube bodies that are joined together, for example glued together, and all tube bodies or just some of the tube bodies can be flocked with the flock material.

In an alternative embodiment, the tube according to the invention can comprise several single, i.e. separately configured, tube bodies. All tube bodies or just some of the tube bodies can be flocked with the flock material.

In a further embodiment, the tube body can be configured as a branched tube body. For example, the tube body can comprise two, three or more branches, with all branches or just some of the branches being flocked with the flock material. Thus, blockage of one branch can be advantageously compensated by the remaining branches without need of interrupting therapy of a patient.

For leading away body fluids or for fluid communication between the surroundings of the tube and a lumen of the tube body it is envisaged in preferred embodiments that the tube body has openings in its wall, especially in the form of holes, perforations, pores, cut-outs or the like. The openings are as a rule configured in the form of through-holes, i.e. as a rule they breach the wall of the tube body. Preferably the tube body has corresponding openings in the region of at least one of its ends, especially in the region of only one of its ends.

Furthermore, the tube body can have protuberances, especially for increasing its surface area.

In another embodiment, the tube body has a circular cross-section or non-circular cross-section, especially an oval, ellipsoidal, crescent-shaped, polygonal for example triangular, rectangular, square, rhombus-shaped, pentagonal, hexagonal or star-shaped cross-section.

In particular, the cross-section of the tube body can be variable over its length.

For example the tube body can be configured as conical or as a tapered section.

In further embodiments, the tube, especially the tube body and/or the flock material, has additives such as in particular physiologically compatible materials, preferably cosmetic, biological and/or medical or pharmaceutical active ingredients, X-ray contrast media, radio-opaque substances, textile auxiliaries such as for example guide aids or spiral mandrin or the like. Regarding suitable physiologically compatible materials or active substances, reference is made entirely to the description so far.

As a supplement or alternative, the tube according to the invention, especially the tube body and/or the flock material, can have cells. Suitable cells can be selected from the group comprising fibroblasts, chondrocytes, osteocytes, osteoblasts, adipocytes, myocytes, neurons, astrocytes, oligodendrocytes, hepatocytes, pancreatic cells, precursor cells thereof, stem cells, especially mesenchymal stem cells, adult stem cells, umbilical cord stem cells and/or fetal stem cells, and mixtures thereof. By using cells or enriched cells, for example the progress of wound healing can be accelerated.

Furthermore, the cells can be autologous, allogenous and/or xenogenous cells. To avoid immune reactions, cells of autologous origin are basically preferred.

To supplement or as an alternative to the embodiment described in the three preceding paragraphs, the tube according to the invention, especially the tube body and/or the flock material, can have platelet-rich plasma (PRP).

In a further embodiment, the tube according to the present invention can be present in a compressed state due to a preferably dissolvable wrapping or impregnation. The wrapping and impregnation, respectively can be formed of a protein such as for example collagen and/or gelatine and/or a polysaccharide such as for example starch and/or hyaluronic acid.

Though sliding properties of the tube according to the present invention can be targeted by choice of the flock material, it can be further envisaged by the present invention that the tube comprises a slide coating. Thus, the sliding properties of the tube can be additionally improved. The slide coating can be formed of a protein and/or polysaccharide, in particular selected from the group comprising collagen, gelatine, starch, hyaluronic acid and combinations thereof.

Regarding further features and advantages of the tube, especially of tube body and/or of flock material, reference is made entirely to the description given hereunder.

In a second aspect, the invention relates to a process for producing a tube such as has been described in the preceding embodiments.
The process comprises the steps:
a) at least partial, especially partial or complete, coating of the surface of at least one tube body with an adhesive and
b) flocking the adhesive-coated tube body with a flock material, while the adhesive is moist, adhesive or sticky, or after activating the adhesive.

The term "flocking" means, in the sense of the present invention, a technique for applying a flock material in the form of fibres on the adhesive layer at least partially covering the surface of the tube body.

The term "activating" or "activate" should comprise, in the sense of the present invention, a technique by means of which the adhesive can be made moist, adhesive or sticky. Corresponding techniques can be selected from the group comprising melting, liquefying, moistening, impregnating, dipping, heating, irradiating, production of ultrasonic waves, the use of chemicals and combinations thereof.

In order to be able to flock the tube body with the maximum possible amount of flock material or to produce a high density of flock material on the tube body, in desirable embodiments a larger tube body is used for producing the tube than the tube bodies described hitherto in the prior art in connection with vacuum therapy. Regarding suitable outside diameters for a tube body for producing the tube according to the invention, reference is made to the outside diameters disclosed in the context of the first aspect of the invention.

In a preferred embodiment, the tube body is provided, before or after flocking, preferably before flocking, with openings, especially in the form of holes, pores, cut-outs, perforations or the like, as a rule with penetration through the tube body wall.

The tube body can be coated with the adhesive for example by extrusion, injection, moulding, screen-printing, dipping, impregnating, spraying, spreading, knife-coating and/or rolling techniques.

The coating techniques described above depend in particular on the adhesive concretely used.

If for example the adhesive is formed from a fusible material, the adhesive can be applied on the tube body by extrusion techniques. If, however, the adhesive is in the form of an aqueous liquid, especially aqueous solution, it may be preferable to apply the adhesive on the tube body by means of a dipping, spraying or spreading technique.

If the adhesive is in the form of paste or gel, especially hydrogel, it may be advantageous to apply the adhesive on the tube body by brushing, spreading, knife-coating, rolling or the like.

The flocking of the tube body can basically be carried out by means of scattering techniques, spraying techniques, blowing techniques, vibration techniques and/or electrostatic techniques.

Preferably the flocking of the tube body is carried out by means of electrostatic flocking.

In electrostatic flocking, the flock material is applied in an electric field on the adhesive layer at least partially covering the surface of the tube body.

In desirable embodiments, electrostatic flocking is carried out in a flocking machine. As a rule the flocking machine receives a negative charge, while as a rule the tube body is grounded. As a result the flock material is usually "shot" perpendicularly or essentially perpendicularly onto the adhesive layer at least partially covering the surface of the tube body.

Owing to the high acceleration that the flock material experiences in the electrostatic field, as a rule good penetration of the flock material into the adhesive layer at least partially covering the surface of the tube body is achieved.

In preferred embodiments the tube is used as electrode, especially grounded electrode, together with a counter-electrode, especially a high-voltage electrode. Especially preferably, the tube body is used as cathode and the counter-electrode, preferably a high-voltage electrode, is used as anode.

The electrodes (tube body and counter-electrode) can be spaced from 2 cm to 200 cm apart, especially 5 cm to 100 cm, preferably 7 cm to 50 cm.

Furthermore, for carrying out electrostatic flocking, a voltage from 5 kV (kilovolt) to 120 kV (kilovolt), especially 10 kV (kilovolt) to 100 kV (kilovolt), preferably 20 kV (kilovolt) to 80 kV (kilovolt), can be applied between the electrodes.

After flocking and especially before a drying step, it can be envisaged according to the invention to modify the flocked tube body, especially the flock material, in order to achieve particular effects. For example, the flocked tube body, especially the flock material, can be modified with respect to orientation, stiffness, shape, cross-section, possible crimping and/or length. This can provide indication-specific and/or patient-specific adaptation of the flock material (and thus of the tube). Corresponding modifications can be achieved for example by means of heat, welding, especially thermal welding, chemicals or the like.

To improve its sliding properties, hydrophilization of the tube, especially of the tube body and/or the flock material, can further be envisaged. Alternatively, to improve its sliding properties the tube can also be lip-ophilized, which can be achieved for example through an appropriate choice of the flock material or (subsequent) modification of the flock material.

Regarding further features and advantages of the process described in the preceding embodiments, especially of the tube, tube body and/or of the flock material, reference is made entirely to the embodiments made in the context of the first aspect of the invention.

In a third and last aspect, the invention relates to a drainage system, especially in the form of a kit. The drainage system is intended in particular for use in vacuum therapy, preferably endoluminal vacuum therapy.

The drainage system is characterized in that it has at least one medical drainage tube as disclosed in the description so far, and additionally at least one further component, wherein the further component is selected from the group comprising sponge, inserting instrument, for example overtube or trocar, source of vacuum or negative pressure, redrop bottle, guide wire, spiral mandrin and combinations thereof.

The medical tube of the present invention is a medical drainage tube.

The use of a sponge may be advantageous for sealing or closing off a body orifice during a drainage operation. Moreover, the sponge can also be flocked, i.e. comprise a flock material, especially a flock material with antibacterial additive. Regarding further features and advantages of the flock material and a possible adhesive layer for joining the flock material to the sponge, reference is made entirely to the description so far.

Regarding further features and advantages of the drainage system, especially of the medical tube, reference is made entirely to the description so far.

The objects discussed in the preceding aspects of the invention (tube, production process and drainage system) can also each be used for topological applications, especially for topological vacuum therapy. Further features and advantages of the invention can be seen from the following description of preferred embodiments in the form of drawings, descriptions of drawings and practical examples. Moreover, individual features can in each case be realized individually or in combination with one another. The preferred embodiments only serve for further explanation and better understanding of the invention, without limiting it to these.

### Description of the drawings

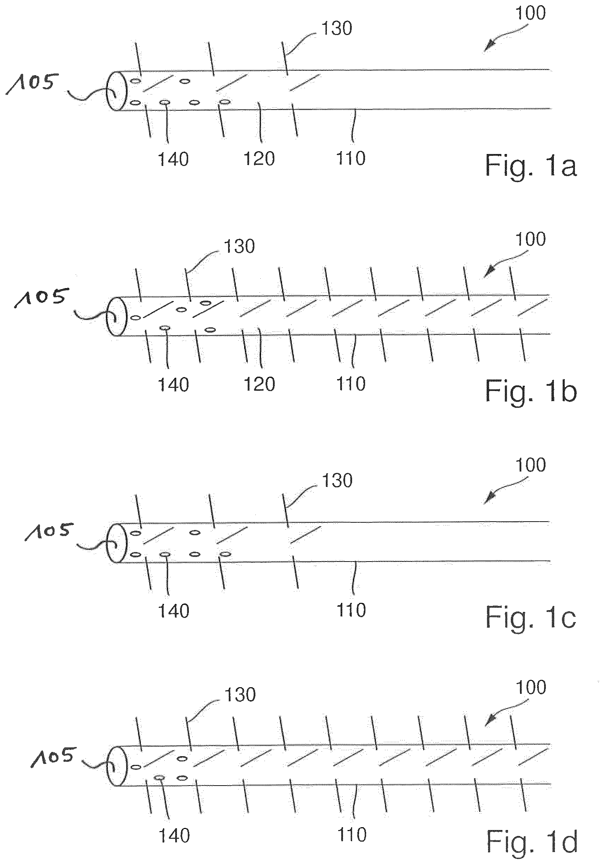

Fig. 1 a shows schematically a medical tube 100 according to the present invention. This comprises a tube body 110, an adhesive layer 120 and a flock material 130 in the form of fibres (flock fibres). The tube body 110 has a lumen (cavity) 105. The flock fibres can for example be formed from a polyamide (nylon flock fibres). Moreover, the flock fibres can for example contain silver as additive for achieving antimicrobial functionality.

The adhesive layer 120 covers the outside surface of the tube body 110 only on one of its two ends. The flock fibres 130 are connected via the adhesive layer 120 to the tube body 110 or its outside surface and protrude from the adhesive layer 120.

For leading away body fluids, especially wound fluids, blood, lymph or the like, the tube body 110 can moreover have openings 140 in the region of its flocked end.

Fig. 1b shows schematically another embodiment of a tube 100 according to the invention. The tube 100 has a tube body 110 with a lumen (cavity) 105, an adhesive layer 120 and a flock material 130 in the form of fibres (flock fibres) and is flocked in its entirety or on the whole area with the flock fibres 130.

In contrast to the tube shown in Fig. 1a, the adhesive layer 120 covers the outside surface of the tube body 110 continuously or on the whole area (apart from optional openings 140). Correspondingly, the tube body 110 or its outside surface is flocked continuously (apart from optional openings 140) with the flock material 130.

The tube 100 shown in Fig. 1b can for example be placed in the form of a "ball" in a body cavity, so that optimum filling of the cavity with the tube 100 can be achieved.

The tubes shown in Figs. 1a and 1b are used as drainage tubes, especially for carrying out vacuum therapy, preferably endoluminal vacuum therapy. In this case it may furthermore be preferable if one of the tube ends, especially the flocked tube end (of the tubes shown in Figs. 1a and 1b), is completely sealed, for example glued or welded. This can, especially advantageously, increase the suction power or the suction capacity of the drainage tubes.

### Examples section

### Example 1:

In the context of a continuous flocking process, an endless tube made of polyurethane was fed at a speed of 1 cm/s through a flocking machine, which had a gluing zone, a sieving-dosing unit, a drying zone and a vacuum zone. In the gluing zone, the polyurethane tube was coated in its entirety at a distance of 100 cm in each case over a tube length of 10 cm by means of a paint brush with an adhesive that is commercially available under the designation Mecoflock L 856. On entering the sieving-dosing unit, the tube segments coated with the adhesive were in each case flocked at a high voltage of 60 kV (kilovolt) with polyamide flock fibres. The distance from the tube segments to be flocked to the sieving-dosing applicator was also 40 cm. The resultant flocked endless tube was dried at a temperature between 60 and 90°C in the drying zone. In the vacuum zone that came next, flock fibres not adhesively bonded were removed by applying a negative pressure or vacuum.

### Example 2:

A tube made of polyvinyl chloride (without drainage holes) was dipped over a tube length of 40 cm several times in a melt of poly-4-hydroxybutanoic acid, until the outside surface of the tube was covered continuously with a uniform layer of poly-4-hydroxybutanoic acid.

To activate the layer of poly-4-hydroxybutanoic acid, a flocking booth was heated by means of infrared lamps to a temperature above 75°C. After the layer of poly-4-hydroxybutanoic acid had been partially fused in this way, the polyvinyl chloride tube was flocked with flock fibres made of polyglycolic acid by applying a high voltage of 60 kV (kilovolt) for 3 minutes. The distance between the flock applicator and the polyvinyl chloride tube was 40 cm. After flocking, the flocking booth was ventilated with cold ambient air. Flock fibres not adhesively bonded were then removed by means of a fan (after a cooling phase of 15 minutes).

Further features and advantages of the invention can be seen from the following description of preferred embodiments in the form of drawings, descriptions of drawings and practical examples. Moreover, individual features can in each case be realized individually or in combination with one another. The preferred embodiments only serve for further explanation and better understanding of the invention, without limiting it to these.

### Description of the drawings

Fig. 1a shows schematically a medical tube 100 according to the present invention. This comprises a tube body 110, an adhesive layer 120 and a flock material 130 in the form of fibres (flock fibres). The tube body 110 has a lumen (cavity) 105. The flock fibres can for example be formed from a polyamide (nylon flock fibres). Moreover, the flock fibres can for example contain silver as additive for achieving antimicrobial functionality.

The adhesive layer 120 covers the outside surface of the tube body 110 only on one of its two ends. The flock fibres 130 are connected via the adhesive layer 120 to the tube body 110 or its outside surface and protrude from the adhesive layer 120.

For leading away body fluids, especially wound fluids, blood, lymph or the like, the tube body 110 can moreover have openings 140 in the region of its flocked end.

Fig. 1b shows schematically another embodiment of a tube 100 according to the invention. The tube 100 has a tube body 110 with a lumen (cavity) 105, an adhesive layer 120 and a flock material 130 in the form of fibres (flock fibres) and is flocked in its entirety or on the whole area with the flock fibres 130.

In contrast to the tube shown in Fig. 1a, the adhesive layer 120 covers the outside surface of the tube body 110 continuously or on the whole area (apart from optional openings 140). Correspondingly, the tube body 110 or its outside surface is flocked continuously (apart from optional openings 140) with the flock material 130.

The tube 100 shown in Fig. 1b can for example be placed in the form of a "ball" in a body cavity, so that optimum filling of the cavity with the tube 100 can be achieved.

Fig. 1c shows schematically another embodiment of a tube 100 according to the invention. This has a tube body 110 with a lumen (cavity) 105 and a flock material 130 in the form of fibres (flock fibres). The fibres 130 can for example be polyurethane fibres.

The fibres 130 are only arranged, in the region of one of the two ends of the tube body 110, on the latter or its outside surface.

For leading away body fluids, especially wound fluids, blood, lymph or the like, the tube body 110 can moreover have openings 140 in the region of its flocked end.

In contrast to the tubes shown in Fig. 1a and 1b, the flock material 130 is connected directly, i.e. without the use of an adhesive layer covering the outside surface of the tube body, to the tube body 110 or its outside surface. Rather, the flock material 130 penetrates directly into the wall of the tube body 110. A tube body suitable for this can be formed for example from polyvinyl chloride or polyurethane.

Fig. 1d shows schematically another embodiment of a tube 100 according to the invention. This has a tube body 110 with a lumen (cavity) 105 and a flock material 130 in the form of fibres (flock fibres), for example in the form of viscose fibres.

For leading away body fluids, the tube body 110 can also have openings 140 in the region of its flocked end.

In contrast to the tube shown in Fig. 1c, the tube body 110 or its outside surface is flocked continuously or on the whole area (apart from possible openings 140) with the flock material 130.

The tube 100 shown in Fig. 1d can also be placed for example in the form of a "ball" in a body cavity, so that optimum filling of the cavity with the tube 100 can be achieved.

The tubes shown in Figs. 1a-d can preferably be used as drainage tubes, especially for carrying out vacuum therapy, preferably endoluminal vacuum therapy. In this case it may furthermore be preferable if one of the tube ends, especially the flocked tube end (of the tubes shown in Figs. 1a-d), is completely sealed, for example glued or welded. This can, especially advantageously, increase the suction power or the suction capacity of the drainage tubes.

Alternatively, as an example not being part of the invention, the tubes shown in Figs. 1a-d can be used for introducing a rinsing fluid, supplying therapeutic agents, monitoring or control of a negative pressure/vacuum and/or for temperature monitoring. In these cases it may be desirable if the tube body does not have any openings, especially in the form of holes, pores, cut-outs or the like.

### Examples section

### Example 1:

The perforated (with holes with a diameter of 1 mm) end of a polyurethane tube with an outside diameter of 5 mm and an inside diameter of 3 mm was dipped over a length of 6 cm in a THF bath for 5 seconds. Then the polyurethane tube solubilized in this way was immediately introduced into a flocking booth and was flocked by applying a high voltage of 60 kV (kilovolt) with polyurethane flock fibres with a length of 5 mm (30 dtex). The polyurethane flock fibres were then applied by means of a sieving-dosing applicator on the outside surface of the polyurethane tube, wherein the sieving-dosing applicator was arranged at a distance of 40 cm from the polyurethane tube. Then the resultant flocked polyurethane tube was dried for 6 hours at a temperature of 80°C. Polyurethane flock fibres that were not adhesively bonded were removed by means of a vacuum suction device.

### Example 2:

In the context of a continuous flocking process, an endless tube made of polyurethane was fed at a speed of 1 cm/s through a flocking machine, which had a gluing zone, a sieving-dosing unit, a drying zone and a vacuum zone. In the gluing zone, the polyurethane tube was coated in its entirety at a distance of 100 cm in each case over a tube length of 10 cm by means of a paint brush with an adhesive that is commercially available under the designation Mecoflock L 856. On entering the sieving-dosing unit, the tube segments coated with the adhesive were in each case flocked at a high voltage of 60 kV (kilovolt) with polyamide flock fibres. The distance from the tube segments to be flocked to the sieving-dosing applicator was also 40 cm. The resultant flocked endless tube was dried at a temperature between 60 and 90°C in the drying zone. In the vacuum zone that came next, flock fibres not adhesively bonded were removed by applying a negative pressure or vacuum.

### Example 3:

A tube made of polyvinyl chloride (without drainage holes) was dipped over a tube length of 40 cm several times in a melt of poly-4-hydroxybutanoic acid, until the outside surface of the tube was covered continuously with a uniform layer of poly-4-hydroxybutanoic acid.

To activate the layer of poly-4-hydroxybutanoic acid, a flocking booth was heated by means of infrared lamps to a temperature above 75°C. After the layer of poly-4-hydroxybutanoic acid had been partially fused in this way, the polyvinyl chloride tube was flocked with flock fibres made of polyglycolic acid by applying a high voltage of 60 kV (kilovolt) for 3 minutes. The distance between the flock applicator and the polyvinyl chloride tube was 40 cm. After flocking, the flocking booth was ventilated with cold ambient air. Flock fibres not adhesively bonded were then removed by means of a fan (after a cooling phase of 15 minutes).

## Claims

1. Medical drainage tube, especially for use in vacuum therapy, preferably endoluminal vacuum therapy, comprising at least one tube body and a flock material, wherein the flock material is joined to the tube body or its surface via an adhesive layer covering the surface of the tube body at least partially and the flock material is flock fibres, **characterized in that** the flock fibres are disposed on only one of the ends of the tube body on the adhesive layer at least partially covering the surface of the tube body.

2. Medical drainage tube according to Claim 1, **characterized in that** the adhesive layer covers the surface of the tube body only partially.

3. Medical drainage tube according to Claim 1 or 2, **characterized in that** the adhesive is selected from the group comprising hot-melt adhesives, especially hot activatable adhesives or hot-melt adhesives, preferably based on a polyamide, polyester, polyolefin, especially amorphous poly-α-olefin, and/or polyurethane, especially thermoplastic polyurethane, polyvinyl alcohol, polyvinyl acetate, polyvinyl chloride, polyvinyl propionate, polyacrylates, cyanoacrylates, modified vinyl acetate copolymers, polyester elastomers, polyurethane elastomers, vinylpyrrolidone/vinyl acetate copolymer, polycarbonates, rubber, acrylonitrile-butadiene-styrene copolymers, hydrogels based on polyethylene glycol, resins such as synthetic resins, waxes, especially synthetic waxes and/or beeswaxes and mixtures thereof.

4. Medical drainage tube according to one of the preceding claims, **characterized in that** the flock material is selected from the group comprising polyolefins, polyamides, polyimides, polyesters, polyurethanes, especially thermoplastic polyurethanes, polyhydroxyalkanoates, proteins, polysaccharides, copolymers thereof, salts thereof, stereoisomers thereof and mixtures thereof, especially from the group consisting of polyethylene, low-density polyethylene, high-density polyethylene, high-molecular polyethylene, ultra-high-molecular polyethylene, polypropylene, polyethylene terephthalate, polypropylene terephthalate, polybutylene terephthalate, polyamide 6, polyamide 6-6, polyamide 6-12, polyamide 12, silk, especially rayon or spider silk, polytetrafluorethylene, polyvinylidene difluoride, polytetrafluoropropylene, polyhexafluoropropylene, polyvinyl alcohol, polyglycolide, polylactide, polydioxanone, poly-3-hydroxybutyrate, poly-4-hydroxybutyrate, polytrimethylene carbonate, poly-ε-caprolactone, collagen, gelatin, elastin, reticulin, fibronectin, laminin, fibrin, fibrinogen, albumin, starch, amylose, amylopectin, dextran, viscose, cotton, cellulose, methylcellulose, carboxymethylcellulose, chitosan, hyaluronic acid, dextran sulphate, heparin, heparan sulphate, chondroitin sulphate, dermatan sulphate, copolymers thereof, salts thereof, stereoisomers thereof and mixtures thereof.

5. Medical drainage tube according to one of the preceding claims, **characterized in that** the tube body has an outside diameter from 1 mm to 15 mm, especially 2 mm to 10 mm, preferably 3 mm to 7 mm.

6. Medical drainage tube according to one of the preceding claims, **characterized in that** the tube body is formed from a material that is selected from the group comprising polyvinyl chloride, polyurethane, polyethylene, polypropylene, silicone and mixtures thereof.

7. Medical drainage tube according to one of the preceding claims, **characterized in that** the tube body is of multilumen configuration.

8. Medical drainage tube according to one of the preceding claims, **characterized in that** the tube body has openings, especially in the form of holes, pores, perforations, cut-outs or the like.

9. Process for producing a medical drainage tube according to one of the preceding claims, comprising the steps:
a) coating the surface of at least one tube body with an adhesive at least partially, especially partially or completely, and
b) flocking the adhesive-coated tube body with a flock material, while the adhesive is moist, adhesive or sticky, or after activating the adhesive.

10. Drainage system, especially in the form of a kit, comprising at least one medical drainage tube according to one of Claims 1 to 8 and at least one further component that is selected from the group comprising sponge, inserting instrument, for example overtube or trocar, source of vacuum or negative pressure, redrop bottle, guide wire, spiral mandrin and combinations thereof.

## Patentansprüche

1. Medizinischer Drainageschlauch, insbesondere zur Verwendung in der Vakuumtherapie, bevorzugt der endoluminalen Vakuumtherapie, umfassend wenigstens einen Schlauchkörper und ein Flockmaterial, wobei das Flockmaterial über eine die Oberfläche des Schlauchkörpers wenigstens teilweise bedeckende Klebstoffschicht mit dem Schlauchkörper bzw. dessen Oberfläche verbunden ist, und es sich bei dem Flockmaterial um Flockfasern handelt, **dadurch gekennzeichnet, dass** die Flockfasern an nur einem der Enden des Schlauchkörpers auf der die Oberfläche des Schlauchkörpers wenigstens teilweise bedeckenden Klebstoffschicht angeordnet sind.

2. Medizinischer Drainageschlauch nach Anspruch 1, **dadurch gekennzeichnet, dass** die Klebstoffschicht die Oberfläche des Schlauchkörpers nur teilweise bedeckt.

3. Medizinischer Drainageschlauch nach Anspruch 1 oder 2, **dadurch gekennzeichnet, dass** der Klebstoff ausgewählt ist aus der Gruppe umfassend Schmelzklebstoffe, insbesondere heiß aktivierbare Klebstoffe bzw. hot-melt Klebstoffe, bevorzugt auf Basis eines Polyamids, Polyesters, Polyolefins, insbesondere amorphen Poly-α-Olefins, und/oder Polyurethans, insbesondere thermoplastischem Polyurethans, Polyvinylalkohol, Polyvinylacetat, Polyvinylchlorid, Polyvinylpropionat, Polyacrylate, Cyanoacrylate, modifizierte Vinylacetatcopolymere, Polyester-Elastomere, Polyurethan-Elastomere, Vinylpyrrolidon-Vinylacetat-Copolymer, Polycarbonate, Gummi, Acrylnitril-Butadien-Styrol-Copolymere, Hydrogele auf Basis von Polyethylenglykol, Harze wie beispielsweise synthetische Harze, Wachse, insbesondere synthetische Wachse und/oder Bienenwachse und Mischungen davon.

4. Medizinischer Drainageschlauch nach einem der vorhergehenden Ansprüche, **dadurch gekennzeichnet, dass** das Flockmaterial ausgewählt ist aus der Gruppe umfassend Polyolefine, Polyamide, Polyimide, Polyester, Polyurethane, insbesondere thermoplastische Polyurethane, Polyhydroxyalkanoate, Proteine, Polysaccharide, Copolymere davon, Salze davon, Stereoisomere davon und Mischungen davon, insbesondere aus der Gruppe bestehend aus Polyethylen, Polyethylen niedriger Dichte, Polyethylen hoher Dichte, hochmolekulares Polyethylen, ultrahochmolekulares Polyethlyen, Polypropylen, Polyethylenterephthalat, Polypropylenterephthalat, Polybutylenterephthalat, Polyamid 6, Polyamid 6-6,
Polyamid 6-12, Polyamid 12, Seide, insbesondere Kunst- oder Spinnenseide, Polytetrafluorethylen, Polyvinylidendifluorid, Polytetrafluorpropylen, Polyhexafluorpropylen, Polyvinylalkohol, Polyglykolid, Polylactid, Polydioxanon, Poly-3-Hydroxybutyrat, Poly-4-Hydroxybutyrat, Polytrimethylencarbonat, Poly-ε-Caprolacton, Kollagen, Gelatine, Elastin, Retikulin, Fibronektin, Laminin, Fibrin, Fibrinogen, Albumin, Stärke, Amylose, Amylopektin, Dextran, Viskose, Baumwolle, Cellulose, Methylcellulose, Carboxymethylcellulose, Chitosan, Hyaluronsäure, Dextransulfat, Heparin, Heparansulfat, Chondroitinsulfat, Dermatansulfat, Copolymere davon, Salze davon, Stereoisomere davon und Mischungen davon.

5. Medizinischer Drainageschlauch nach einem der vorhergehenden Ansprüche, **dadurch gekennzeichnet, dass** der Schlauchkörper einen Außendurchmesser von 1 mm bis 15 mm, insbesondere 2 mm bis 10 mm, bevorzugt 3 mm bis 7 mm, aufweist.

6. Medizinischer Drainageschlauch nach einem der vorhergehenden Ansprüche, **dadurch gekennzeichnet, dass** der Schlauchkörper aus einem Material gebildet ist, das ausgewählt ist aus der Gruppe umfassend Polyvinylchlorid, Polyurethan, Polyethylen, Polypropylen, Silikon und Mischungen davon.

7. Medizinischer Drainageschlauch nach einem der vorhergehenden Ansprüche, **dadurch gekennzeichnet, dass** der Schlauchkörper eine Multilumen-Konfiguration aufweist.

8. Medizinischer Drainageschlauch nach einem der vorhergehenden Ansprüche, **dadurch gekennzeichnet, dass** der Schlauchkörper Öffnungen, insbesondere in Form von Löchern, Poren, Perforationen, Einschnitten oder dergleichen, aufweist.

9. Verfahren zur Herstellung eines medizinischen Drainageschlauches nach einem der vorhergehenden Ansprüche, umfassend die Schritte:
a) wenigstens teilweises, insbesondere teilweises oder vollständiges, Beschichten der Oberfläche wenigstens eines Schlauchkörpers mit einem Klebstoff und
b) Beflocken des mit Klebstoff beschichteten Schlauchkörpers mit einem Flockmaterial, solange der Klebstoff feucht, adhäsiv bzw. klebrig ist, oder nach Aktivieren des Klebstoffes.

10. Drainagesystem, insbesondere in Form eines Kits, umfassend wenigstens einen medizinischen Drainageschlauch nach einem der Ansprüche 1 bis 8 und wenigstens eine weitere Komponente, die ausgewählt ist aus der Gruppe umfassend Schwamm, Einführinstrument, beispielsweise Overtube oder Trokar, Vakuum- oder Unterdruckquelle, Redrop-Flasche, Führungsdraht, Spiralmandrin und Kombinationen davon.

## Revendications

1. Tube de drainage médical, destiné en particulier à être utilisé lors d'une thérapie par le vide, de préférence lors d'une thérapie endoluminale par le vide, comportant au moins un corps de tube et un matériau de flocage, dans lequel le matériau de flocage est joint au corps de tube ou sa surface par le biais d'une couche adhésive recouvrant la surface du corps de tube au moins partiellement et le matériau de flocage est composé de fibres de flocage, **caractérisé en ce que** les fibres de flocage sont disposées sur seulement une des extrémités du corps de tube sur la couche adhésive recouvrant au moins partiellement la surface du corps de tube.

2. Tube de drainage médical selon la revendication 1, **caractérisé en ce que** la couche adhésive recouvre la surface du corps de tube seulement partiellement.

3. Tube de drainage médical selon la revendication 1 ou 2, **caractérisé en ce que** l'adhésif est sélectionné dans le groupe constitué par les adhésifs thermofusibles, en particulier les adhésifs thermo-activables ou les adhésifs thermofusibles, de préférence à base d'un polyamide, un polyester, une polyoléfine, particulièrement une poly-α-oléfine amorphe, et/ou un polyuréthane, particulièrement un polyuréthane thermoplastique, un alcool polyvinylique, un acétate polyvinylique, un chlorure polyvinylique, un propionate polyvinylique, des polyacrylates, des cyanoacrylates, un copolymère d'acétate de vinyle modifié, des élastomères de polyester, des élastomères de polyuréthane, des copolymères de vinyle pyrrolidone/vinyle acétate, des polycarbonates, du caoutchouc, des copolymères d'acrylonitrile-butadiène-styrène, des hydrogels à base de polyéthylène glycol, de résines telles que les résines synthétiques, les cires, particulièrement les cires synthétiques et/ou les cires d'abeille ou leurs mélanges.

4. Tube de drainage médical selon l'une quelconque des revendications précédentes, **caractérisé en ce que** le matériau de flocage est sélectionné dans le groupe constitué par les polyoléfines, les polyamides, les polyimides, les polyesters, les polyuréthanes, en particulier les polyuréthanes thermoplastiques, les polyhydroxyalcanoates, les protéines, les polysaccharides, leurs copolymères, leurs sels, leurs stéréoisomères et leurs mélanges, en particulier dans le groupe constitué par le polyéthylène, le polyéthylène de faible densité, le polyéthylène de haute densité, le polyéthylène de poids moléculaire élevé, le polyéthylène de poids moléculaire très élevé, le polypropylène, le téréphtalate de polyéthylène, le téréphtalate de polypropylène, le téréphtalate de polybutylène, le polyamide 6, le polyamide 6-6, le polyamide 6-12, le polyamide 12, la soie, en particulier la rayonne ou la soie d'araignée, le polytétrafluoréthylène, le difluorure de polyvinylidène, le polytétrafluoropropylène, le polyhexafluoropropylène, l'alcool polyvinylique, le polyglycolide, le polylactide, la polydioxanone, le poly(3-hydroxybutyrate), le poly(4-hydroxybutyrate), le carbonate de polytriméthylène, la poly(ε-caprolactone), le collagène, la gélatine, l'élastine, la réticuline, la fibronectine, la laminine, la fibrine, le fibrinogène, l'albumine, l'amidon, l'amylose, l'amylopectine, le dextrane, la viscose, le coton, la cellulose, la méthylcellulose, la carboxyméthylcellulose, le chitosane, l'acide hyaluronique, le sulfate de dextrane, l'héparine, le sulfate d'héparane, le sulfate de chondroïtine, le sulfate de dermatane, leurs copolymères, leurs sels, leurs stéréoisomères et leurs mélanges.

5. Tube de drainage médical selon l'une quelconque des revendications précédentes, **caractérisé en ce que** le corps de tube a un diamètre extérieur compris entre 1 et 15 mm, en particulier entre 2 et 10 mm, de préférence entre 3 et 7 mm.

6. Tube de drainage médical selon l'une quelconque des revendications précédentes, **caractérisé en ce que** le corps de tube est formé à partir d'un matériau qui est sélectionné dans le groupe constitué par le chlorure de polyvinyle, le polyuréthane, le polyéthylène, le polypropylène, la silicone et leurs mélanges.

7. Tube de drainage médical selon l'une quelconque des revendications précédentes, **caractérisé en ce que** le corps de tube a une configuration à lumières multiples.

8. Tube de drainage médical selon l'une quelconque des revendications précédentes, **caractérisé en ce que** le corps de tube présente des ouvertures, particulièrement sous forme de trous, pores, perforations, découpes ou ouvertures similaires.

9. Procédé de production d'un tube de drainage médical selon l'une quelconque des revendications précédentes, comportant les étapes consistant à :
a) recouvrir la surface d'au moins un corps de tube d'un adhésif au moins partiellement, en particulier partiellement ou complètement, et
b) floquer le corps de tube recouvert d'adhésif avec un matériau de flocage, pendant que l'adhésif est humide, adhésif ou collant, ou après l'activation de l'adhésif.

10. Système de drainage, en particulier sous forme de trousse, comportant au moins un tube de drainage médical selon l'une quelconque des revendications 1 à 8, et au moins un composant supplémentaire qui est sélectionné dans le groupe constitué par une éponge, un instrument d'insertion, par exemple un surtube ou un trocart, une source de vide ou de pression négative, une bouteille de rechute, un fil de guidage, un mandrin spiralé et leurs combinaisons.
